# EUROPEAN PATENT APPLICATION

(11) **EP 4 400 044 A1**
(43) Date of publication of application: **17.07.2024**
(21) Application number: 23305034.3
(22) Date of filing: 11.01.2023
(51) Int. Cl.: A61B 5/113, A61B 5/00, A61B 5/01, A61B 5/11

(54) **WEARABLE ARTICLE INCLUDING AN ENCAPSULATED ELONGATION SENSOR**

(71) Applicant: CHRONOLIFE, 75012 Paris (FR)
(72) Inventor: Roger, Harmony, 77173 CHEVRY COSSIGNY (FR); Rémond, Ludovic, 28130 PIERRES (FR); ROMAO, Fernando, 75016 PARIS (FR)
(74) Representative: Plasseraud IP

(57) **Abstract**

The disclosure refers to a wearable article (201), comprising at least:
- a flexible support (212);
- an elastic strap (206) attached to the flexible support (212) by first adhesive bond (113) having elastic properties; and
- an elongation sensor (102) sandwiched between the flexible support (212) and the elastic strap (206);
wherein the elongation sensor (102) is attached to the flexible support (212) and/or to the elastic strap (206) by second adhesive bond (114) present along the elongation sensor (102), and
wherein the elongation sensor (102) has electrically conductive properties and is configured to detect, when the wearable article (201) is pressed against a body of a user wearing the wearable article (201), a physiological parameter of the user that is related to an elongation of the elongation sensor (102).

## Description

The present disclosure generally relates to the field of physiological sensors in a garment, and, in particular, relates to a wearable article comprising an elongation sensor with a protective encapsulation.

### BACKGROUND OF THE INVENTION

Electronic devices for detecting and processing biometric signals of a user have been developed that allow medical supervision of people during their usual activities or situations. Sensors may be applied to the skin or a specific body area, for example the chest, of a user so as to perform temperature measurements, to determine physiological parameters or to detect electrical signals related for example to a cardiac function. Monitoring the various signals delivered by these sensors allows determining a specific physiological condition of a user that might be impaired. For example, when an individual is having a seizure, specific signal features appear on the signals corresponding to the electrocardiogram (ECG) or to respiration.

A convenient possibility to permanently monitor respiratory activity is to integrate a breathing sensor into a garment or some other wearable article. Such a breathing sensor may acquire information about parameters such as frequency and amplitude of breathing of a user and transmit it to external medical equipment, such as monitoring, diagnostic, or stimulating devices.

It is known that an elongation sensor configured to detect breathing of a user may be installed in a flexible sheath disposed on the inner side of a T-Shirt, in such a way that the elongation sensor can freely glide within the sheath.

However, such an arrangement is fragile, and the quality of the flexible sheath and the elongation sensor may be rapidly degraded, in particular when washing the T-Shirt only once or few times. The sheath may not be sufficiently stable and robust to withstand washing of the garment.

WO 2021/048149 A1 discloses an elongation sensor for detecting breathing of a user, and a garment comprising such an elongation sensor. The elongation sensor can freely glide within a sheath disposed on the inner side of the garment.

### SUMMARY OF THE INVENTION

A wearable article is disclosed, comprising at least:
- a flexible support;
- an elastic strap attached to the flexible support by first adhesive bond having elastic properties; and
- an elongation sensor sandwiched between the flexible support and the elastic strap.

The elongation sensor may be attached to the flexible support and/or to the elastic strap by second adhesive bond present along the elongation sensor.

The elongation sensor may have electrically conductive properties. The elongation sensor may be configured to detect, when the wearable article is pressed against a body of a user wearing the wearable article, a physiological parameter of the user. The physiological parameter may be related to an elongation of the elongation sensor.

The flexible support and the elastic strap may provide a flexible and robust encapsulation for the elongation sensor. The elongation sensor may not be degraded even after several dozens of washing cycles of the wearable article.

The elongation sensor may be a stretchable yarn whose electrically conductive properties vary when being stretched or relaxed.

The flexible support may press the elongation sensor against the body of the user. The flexible support may be a clothing material, for example a non-conductive, flexible fabric. The flexible support may also be a second elastic strap.

The physiological parameter may be a parameter related to breathing, such as breathing amplitude or breathing frequency. The physiological parameter may also be related to muscle contraction or relaxation. For example, a length of the elongation sensor may vary when the user breathes or when muscles of the user are contracted or relaxed.

The elongation sensor may be attached to the flexible support and the elastic strap not only at one or several discrete points. The elongation sensor may be attached to the flexible support and the elastic strap by second adhesive bond continuously along a longitudinal direction of the elongation sensor.

The wearable article as a whole may be reversibly stretched or relaxed.

The first adhesive bond and/or second adhesive bond may comprise an adhesive material. The adhesive material may form the first adhesive bond and the second adhesive bond. The adhesive material may be disposed between the flexible support and the elastic strap. According to special embodiment, the elongation sensor may be embedded in said adhesive material (i.e. example of second adhesive bond).

The adhesive material may provide a matrix in which the elongation sensor is embedded. The elastic strap and the flexible support may be attached to the matrix on opposite sides.

In an embodiment, the adhesive material may have elastic properties, in such a way that an elongation of the elongation sensor generates an elongation of the adhesive material.

The adhesive material may be an elastic material with adhesive properties that allows reversible stretching and relaxing of the flexible support and the elastic strap.

In a particular embodiment, the first adhesive bond and/or second adhesive bond may be created by applying heat to the flexible support and to the elastic strap, with or without using an additional adhesive material.

In such an embodiment, the first adhesive bond and/or the second adhesive bond may be created by heat-bonding.

Heat-bonding allows assembling different materials by applying heat and pressure to them. Heat-bonding allows forming a tight, stable and flexible connection between the flexible support, the elastic strap and optionally the elongation sensor.

In an embodiment, the elongation sensor may comprise a body of flexible material. Conductive particles may be embedded in the body of flexible material.

The flexible material may be a non-conductive material that can be bent or stretched without breaking. The conductive particles may be particles that confer electrically conductive properties to a non-conductive material when embedded in such a material. The mechanical and electrical properties of the elongation sensor may be tailored by the choice of the flexible material and/or the conductive particles.

In an embodiment, the wearable article may be a garment comprising a clothing material. The clothing material may cover a portion of a body of the user. The garment may refer to an item of clothing or apparel. The garment may be a top. The top may be a shirt, t-shirt, blouse, sweater, dress, underwear, athletic clothing, swimwear, belt, jacket/coat, or vest.

The clothing material may be stretched when worn by a user and press the elongation sensor against the body of the user, in such a way that a length of the elongation sensor varies when the physiological parameter varies.

In an embodiment, the garment may be chosen in a group consisting of a T-Shirt, a belt and a brassiere.

The integration of elongation sensors into a T-Shirt or a brassiere may allow studying the physiological parameter over many hours, as long as the T-Shirt or the brassiere is worn.

In an embodiment, the elongation sensor may be configured to be pre-stressed when the wearable article is worn by the user.

The elongation sensor may have elasticity and be pre-stressed when the wearable article is worn, with respect to a rest state of the elongation sensor when the wearable article is not worn. Thus, the elongation sensor may be pressed against the body of the user, and even small changes in the physiological parameters may be detected.

In an embodiment, the wearable article may comprise an elastic belt. The elongation sensor may be located in the elastic belt.

The elastic belt may allow attaching the wearable article to the body of the user and press the elongation sensor against the body, in such a way that a length of the elongation sensor varies when the physiological parameter varies.

In an embodiment, the wearable article may comprise an electronic board. The elongation sensor may be connected to the electronic board.

The connection of the elongation sensor to the electronic board may be direct or indirect. The electronic board may be configured to detect variations of electrically conductive properties of the elongation sensor and deduce an elongation from it. For example, breathing amplitude or breathing frequency of a user wearing the wearable article may be deduced.

In an embodiment, the wearable article may comprise a waterproof housing. The waterproof housing may be configured to receive at least the electronic board.

The waterproof housing may provide a waterproof, protective encapsulation for the electronic board.

In an embodiment, the elongation sensor may have a loop portion at a distance from the electronic board, a first section extending from the electronic board to the loop portion and a second section extending from the loop portion back to the electronic board.

The first section and second sections may be portions of the elongation sensor extending away from a point where the elongation sensor is connected directly or indirectly to the electronic board.

The elongation sensor may extend, starting from a first point where it is connected (directly or indirectly) to the electronic board, away from the electronic board, then form a loop, and extend further back to the electronic board to a second point where it is connected (directly or indirectly) to the electronic board. The elongation sensor may be "U"-shaped.

This "U-shaped" configuration of the elongation sensor may be useful in a wearable article when the elongation sensor is intended to surround only a portion of the circumference of a body part of the user.

In an embodiment, the elongation sensor may be connected to the electronic board by at least one conductive yarn.

The conductive yarn may be a yarn having electrically conductive properties. The conductive yarn may be configured to transmit changes in the electrically conductive properties of the elongation sensor (for example to an electronic board for further analysis).

In an embodiment, the wearable article may comprise a knotted portion. The elongation sensor may be knotted to the at least one conductive yarn in the knotted portion.

The knotted portion formed by the conductive yarn and the elongation sensor may establish a stable, robust, compact and electrically conductive connection between the conductive yarn and the elongation sensor.

In an embodiment, the elongation sensor may be disposed to be located around at least part of an abdomen and/or a chest of the user.

For example, during breathing, the change of the circumference of the abdomen and the chest may be more important when compared to other body parts. Elongation sensors located around the chest/the abdomen allow precise determination of breathing parameters of a user.

### BRIEF DESCRIPTION OF THE DRAWINGS

The accompanying drawings, which are incorporated herein and form a part of the specification, illustrate the present invention and, together with the description, further serve to explain the features of the invention and to enable a person skilled in the pertinent art to make and use the invention. In the drawings, like reference characters indicate identical or functionally similar elements.
- Figure 1 is a schematic representation of the front side of an example of a wearable article according to the invention, the wearable article being a brassiere comprising two elongation sensors;
- Figures 2 is a schematic representation of the front side of another example of a wearable article according to the invention, the wearable article being a T-Shirt comprising an elongation sensor;
- Figures 3 is a schematic representation of the back side of the T-Shirt of Fig. 2;
- Figure 4 is a schematic representation of an arrangement of the elongation sensor, of two conductive yarns, and of two triple knots formed by each of the two conductive yarns with the elongation sensor in the T-Shirt of Fig. 2 and Fig. 3;
- Figure 5 is a partial, schematic cross-sectional view of an example of the T-Shirt of Fig. 2;
- Figure 6 is a partial, schematic cross-sectional view of an elongation sensor sandwiched between a flexible support and an elastic strap in the wearable article of Fig. 2, Fig. 3 or Fig. 5;
- Figure 7 is a flow chart of a method for fabricating a wearable article of Figure 6.

### DESCRIPTION OF PREFERRED EMBODIMENTS

Now, a wearable article that includes physiological sensors for detecting physiological signals of the body of a user wearing the wearable article will be described.

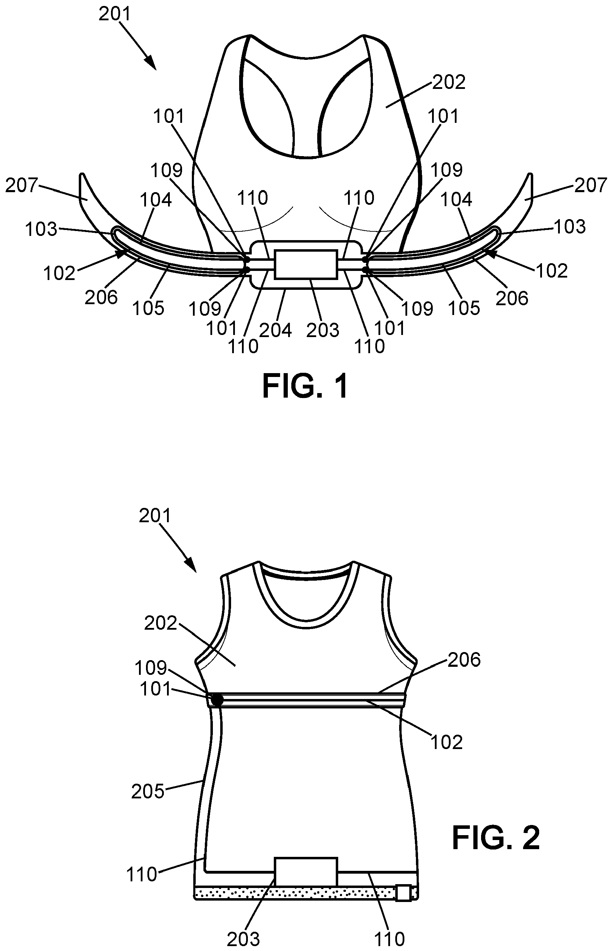

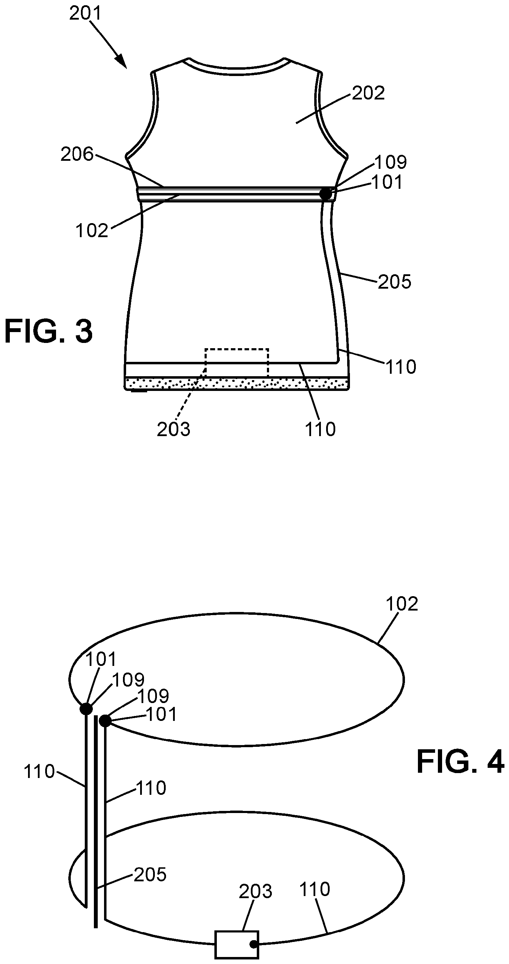

The wearable article 201 may be a brassiere, as shown in figure 1, or a T-Shirt, as shown in figures 2 and 3. The wearable article 201 comprises at least one elongation sensor 102 that has electrically conductive properties. The elongation sensor 102 is configured to detect, when the wearable article 201 is worn by a user and pressed against a body of the user, a physiological parameter such as breathing rate. The elongation of the elongation sensor 102 varies in dependence on the physiological parameter.

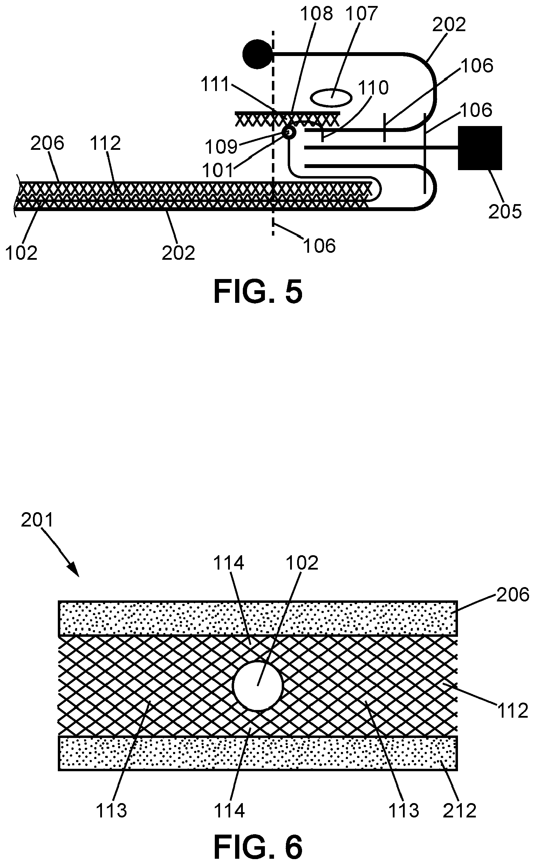

As illustrated in figure 6, the elongation sensor 102 is sandwiched between a flexible support 212 of the wearable article 201 and an elastic strap 206. According to specific embodiment, the flexible support 212 may be a second elastic strap. According to another specific embodiment, the flexible support 212 may be a clothing material 202 covering a portion of the body of the user. The elastic strap 206 is attached to the flexible support 212 by a first adhesive bond 113. The elongation sensor is attached to the flexible support 212 and to the elastic strap 206 by a second adhesive bond 114 along a longitudinal direction of the elongation sensor 102. According to this special embodiment, the elongation sensor 102 may be embedded in the adhesive material forming the first adhesive bond 113 and the second adhesive bond 114.

In the non-limiting embodiment shown in figure 1, the wearable article 201 is a brassiere that includes sensors to detect multiple signals related to heart activity, breathing or body temperature of a user.

The brassiere comprises in this example two elongation sensors 102.

Each elongation sensor 102, which may also be called breathing wire, is a flexible yarn that comprises a body of flexible material such as silicone with conductive particles embedded therein, for example carbon particles. Each elongation sensor 102 has electrically conductive properties that vary as a function of elongation of the elongation sensor 102, i.e. when the user breathes. According to preferred embodiment, the body of flexible material of the elongation sensor 102 has a protective cover made for example with solvron^{®} threads wrapped around it. This protective cover facilitates manipulation of the elongation sensor 102.

The elongation sensor 102 has a resistance of the order of kΩ, for example a resistance comprised between 100 kΩ/m and 200 kΩ/m.

According to special embodiment, the elongation sensor has in a rest state a resistance of 136 kΩ/m when 5 g of carbon particles are used in relation to 1000 g of silicone. According to another embodiment, the elongation sensor has in a rest state a resistance of 165 kΩ/m when 50 g of carbon particles are used in relation to 1000 g of silicone.

The brassiere comprises a clothing material 202 such as polyester covering parts of the chest and the back of a user, and further the shoulders in the form of shoulder straps. The brassiere further comprises a waterproof housing 204 that receives an electronic board 203 configured to collect and process signals obtained from the elongation sensors 102.

The waterproof housing 204 is made from polyester plates that are soldered to each other on their respective edges. According to special embodiment, the waterproof housing 204 may be as disclosed in WO2021028223.

The brassiere also comprises two elastic belts 207, each elongation sensor 102 being located in an elastic belt 207. The waterproof housing 204 is connected to two elastic belts 207 that are attached to opposite sides of the waterproof housing 204. When the brassiere is worn, the waterproof housing 204 is located at the sternum of the user, and the elastic belts 207 extend around a part of the torso of the user. The elastic belts 207 can be linked to each other at the back of the user.

The clothing material 202 of the brassiere is connected to the waterproof housing 204. Therefore, a bottom portion of the clothing material 202 of the brassiere is folded and sewn in order to create a pocket (not shown in figure 1 for clarity reasons) that receives the waterproof housing 204. A portion of the elastic belts 207 protrudes from the pocket in opposite directions.

Each elongation sensor 102 is connected to the electronic board 203 by conductive yarn 110. More precisely, two conductive yarns 110 per elongation sensor 102 are used to connect each elongation sensor 102 indirectly to the electronic board 203. The conductive yarns 110 are sewn to a conductive edge portion with holes of the electronic board 203.

The elongation sensor 102 is knotted to each of the two conductive yarns 110 in a respective knotted portion 101 in order to create an electrical connection between the respective conductive yarn 110 and the elongation sensor 102. Each knotted portion 101 comprises a triple knot 109, i.e. three individual knots made one above the other. The brassiere of figure 1 comprises four such triple knots 109 (two triple knots 109 for each of the two elongation sensors 102). The resulting triple knot 109 is stabilized and protected against water by covering it with an isolating wrapping layer. The isolating wrapping layer is attached to the triple knot 109 by heat bonding.

In this example, each conductive yarn 110 is built from two assembled *Circuitex* silver yarns commercialized by company *Noble Biomaterials.* Each *Circuitex* resistive element has a flexible polyamide core plated with silver. Each conductive yarn 110 corresponding to the two assembled *Circuitex* silver yarns has a resistance of 172 Ω/m. Each conductive yarn 110 is further covered by two *Solvron*^{®} threads to reinforce the conductive yarn 110 and to limit its unravelling.

In this example, the resistance of the conductive yarn 110 is much smaller than the resistance of the elongation sensor 102. Thus, changes of the electrically conductive properties of the conductive yarn 110 may be small and negligible when compared to relative changes of the electrically conductive properties of the elongation sensor 102.

In this example, each elongation sensor 102 forms a loop portion 103 at a distance from the electronic board 203, i.e. the elongation sensor 102 is "U"-shaped. More precisely, a first section 104 of the elongation sensor 102 extends from a first triple knot 109 with one conductive yarn 110 to the loop portion 103, and a second section 105 of the elongation sensor extends from the loop portion 103 back to a second triple knot 109 formed with another conductive yarn 110.

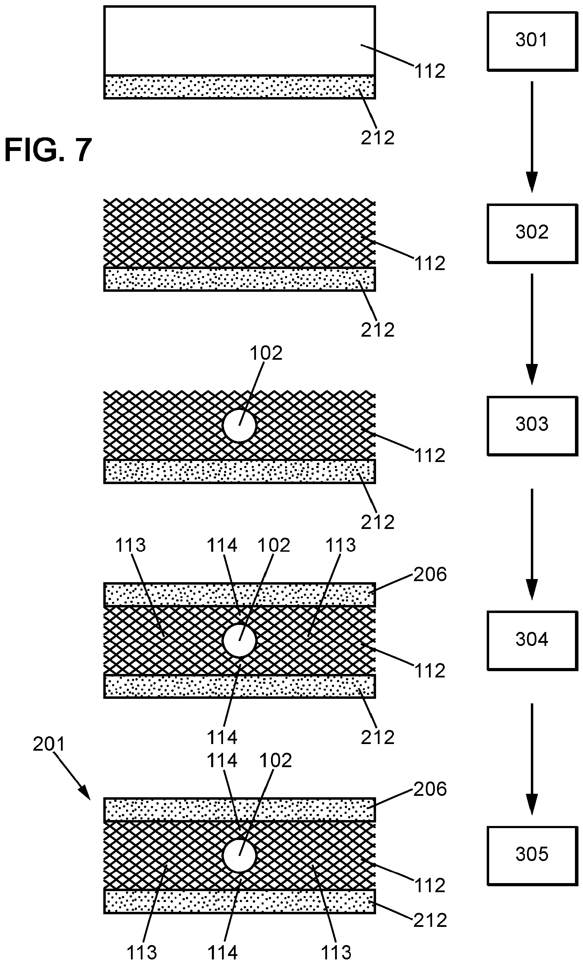

The loop portions 103 of the elongation sensors 102 are located in the elastic belts 207. Each elongation sensor 102 is sandwiched between an elastic strap 206 and a second elastic strap. The elastic strap 206 and the second elastic strap are attached to a respective elongation sensor 102 by first and second adhesive bonds 113, 114, as discussed in relation to figures 6 and 7.

When the elastic belts 207 are tightened to the torso of the user, the elastic belts 207 press the elongation sensors 102 against the torso, such that a length of the elongation sensor 102 varies when the user breathes or when muscles of the user are contracted or relaxed.

The advantage of having two elongation sensors 102 in the brassiere rather than one single elongation sensor 102 is evident when the user wearing the brassiere lays on one side. One elongation sensor 102 may then be squeezed and not work properly. However, the other elongation sensor 102 is still functional.

The electrical connection path from the elongation sensors 102 to the electronic board 203 has a waterproof encapsulation. Therefore, the conductive yarn 110 is encapsulated by elastic tape attached to the conductive yarn 110 by heat bonding.

The electronic board 203 and a portion of the conductive yarn 110 connected to the electronic board 203 is encapsulated by a silicone matrix in order to avoid intrusion of water into the electronic board 203. The encapsulation by a silicone matrix provides an additional protection for the electronic board 203, in addition to the placing of the electronic board 203 in the waterproof housing 204.

The conductive yarn 110 passes from the electronic board 203 through small orifices in the polyester plates of the waterproof housing 204 and further to the elongation sensor 102. The orifices in the polyester plates are sealed by elastic tape attached to the polyester plates by heat bonding, in order to ensure that the space enclosed by the polyester plates is waterproof.

In another non-limiting embodiment shown in figures 2-5, the wearable article 201 is a T-Shirt that includes sensors to detect signals related to heart activity, breathing or body temperature of a user.

The T-Shirt comprises one elongation sensor 102 surrounding the chest of the user. The T-Shirt comprises a flexible clothing material 202 such as elastane cotton having elasticity and covering the chest, back, abdomen and upper arms of the user. The flexible clothing material 202 is stretched when worn by a user, thus pressing the elongation sensor 102 of the T-Shirt against the body of the user such that a length of the elongation sensor 102 varies when the user breathes or when muscles of the user are contracted or relaxed.

The T-Shirt comprises a lateral zipper 205, allowing opening and closing the T-Shirt similarly to a jacket. The zipper 205 makes it easy for a user to put the T-Shirt on and off.

The T-Shirt further comprises an electronic board 203 configured to collect and process signals obtained from the elongation sensors 102. The electronic board 203 is located in the region of the navel of a user.

The elongation sensor 102 has two portions connected to the electronic board 203 by two conductive yarns 110. More precisely, the elongation sensor 102 forms one triple knot 109 in a knotted portion 101 with one conductive yarn 110 connected to the electronic board 203, and another triple knot 109 in another knotted portion 101 with another conductive yarn 110 connected to the electronic board 203. Thus, the T-Shirt comprises two such triple knots 109.

Each resulting triple knot 109 is stabilized and protected against water and wear by covering it with an isolating wrapping layer 108. The isolating wrapping layer 108 is attached to the triple knot 109 by heat bonding by using an adhesive material 111. More precisely, the isolating wrapping layer 108 is made from a sheet of flexible, isolating material that is disposed around the triple knot 109. When applying heat to the sheet, the flexible, isolating material melts and covers the triple knot 109. Having cooled down to room temperature, the flexible, isolating material is attached to the triple knot 109 and forms a stable, waterproof protection for the triple knot 109.

The specific example of figures 2 and 3 and in particular of figure 4 illustrate the path of the elongation sensor 102 and of the two conductive yarns 110 in the T-Shirt. Each of these two conductive yarns 110 are attached to opposite sides of the electronic board 203. The conductive yarns 110 are guided from the electronic board 203 in opposite directions parallel to the bottom edge of the T-Shirt up to the zipper 205. Thus, the conductive yarn 110 almost entirely surrounds the abdomen. Both conductive yarns 110 are then guided along the zipper 205 (and on opposite sides of the zipper 205) up to the chest region.

The elongation sensor 102 is connected to one conductive yarn 110 close to one side of the zipper 205, surrounds the chest and is connected to the other conductive yarn 110 close to the other side of the zipper 205.

A similar arrangement not shown in the figures is provided for the elongation sensor 102 surrounding the abdomen of the user.

In the specific example of the T-Shirt shown in figure 5, stitches 106 are used in the T-Shirt to fix the elongation sensor 102 to the clothing material 202 in a region close to the knotted portion 101, in order to reduce forces acting on the knotted portion 101 when the elongation sensor 102 is stretched. Furthermore, other sensors such as a temperature sensor 107 may be included into the T-Shirt.

A skilled person would understand that a T-Shirt without a zipper is within the scope of the disclosure. When no zipper is present, the elongation sensors 102 surround the chest (or the abdomen) of the user and are indirectly connected to the electronic board 203 by conductive yarn 110. In this example, the conductive yarn 110 does not surround the abdomen.

The conductive yarn 110 is attached to the clothing material 202 of the T-Shirt along its path from the electronic board 203 to the elongation sensor 102.

The electrical connection path from the elongation sensors 102 to the electronic board 203 has a waterproof encapsulation. Therefore, the conductive yarn 110 is encapsulated by elastic tape attached to the conductive yarn 110 by heat bonding. The electronic board 203 and a portion of the conductive yarn 110 connected 110 to the electronic board 203 is encapsulated by a silicone matrix in order to avoid intrusion of water into the electronic board 203.

The elongation sensor 102 is sandwiched between the clothing material 202 (flexible support) of the T-Shirt and an elastic strap 206 attached to the inner side of the clothing material 202 of the T-Shirt by use of first and second adhesive bonds 113, 114. In particular, the elongation sensor 102 is embedded in an adhesive material 112, as discussed in relation to figures 6 and 7.

Figure 6 shows a cross-sectional view of a portion of the wearable article 201. The elongation sensor 102 is sandwiched between the elastic strap 206 and a flexible support 212.

In one specific example, the flexible support 212 comprises the clothing material 202. Indeed, for the case of the T-Shirt, the elongation sensor 102 is sandwiched between the clothing material 202 of the T-Shirt and an elastic strap 206 attached to the clothing material 202 of the T-Shirt.

In another specific example, the flexible support 212 comprises a second elastic strap. Indeed, for the case of the brassiere, the elongation sensor 102 (located in the elastic belts 207) is sandwiched between the elastic strap 206 and the second elastic strap.

The elongation sensor 102 is embedded in a matrix of adhesive material 112 disposed between the elastic strap 206 and the flexible support 212. The adhesive material 112 attaches the flexible support 212, the elastic strap 206 and the elongation sensor 102 to each other, at least along the longitudinal direction of the elongation sensor.

The adhesive material 112 has elastic and flexible properties, allowing extension and bending of the elongation sensor 102 and of the adhesive material 112. When the wearable article 201 is worn, the elongation sensor 102 is pre-stressed (with respect to a rest state when the wearable article 201 is not worn). The elongation sensor 102 is pressed against the body of a user.

The matrix of adhesive material 112 provides a waterproof, robust and flexible encapsulation for the elongation sensor 102 and will not be degraded even after several dozens of washing cycles of the wearable article 201.

Figure 7 is a flow-chart of a method for fabricating the wearable article 201 by heat-bonding.

The adhesive material 112 is a double-sided adhesive scotch tape provided on a substrate.

First, the scotch tape is removed from its substrate and deposited 301 on the flexible support 212. According to special embodiment, said flexible support 212 can be for example a clothing material 202 or a second elastic strap.

Heat is then applied 302 in order to melt the scotch tape. The resulting liquid adhesive material 112 penetrates into the flexible support 212. The elongation sensor 102 is then disposed 303 in the liquid adhesive material 112.

An elastic strap 206 is disposed 304 on top of the adhesive material 112 and the elongation sensor 102. When further applying 305 heat, the adhesive material 112 will penetrate into the elastic strap 206.

Having cooled down to room temperature, the flexible support 212 and the elastic strap 206 are attached to each other by means of the adhesive material 112. The elongation sensor 102 is located between the flexible support 212 and the elastic strap 206 and surrounded by the adhesive material 112.

During the mounting of the elongation sensor 102 and the elastic strap 206 on the clothing material 202 of the T-Shirt by heat-bonding, the portion of the clothing material 202 on which the elongation sensor 102 and the elastic strap 206 are mounted is flat and in its rest state, i.e. it is not pre-stretched. During the mounting, the elongation sensor 102 and the elastic strap 206 are pre-stretched by 15% with respect to their respective rest states.

The T-Shirt fits tightly when being worn by the user, in such a way that the clothing material 202 is stretched with respect to its rest state, and that the elongation sensor 102 and the elastic strap 206 are further stretched with respect to the above-mentioned pre-stretched states. When the T-Shirt is worn, the elongation sensor 102 and the elastic strap 206 may be stretched by up to 30% with respect to their respective rest states, in dependence on the morphology of the user.

When the user breathes normally, the elongation of the elongation sensor 102 varies by approximately 1% during one breathing cycle. When the user breathes heavily, the elongation of the sensor 102 may vary by up to 6% during one breathing cycle.

It will be appreciated that the embodiments described above are illustrative of the invention disclosed herein and that various modifications can be made without departing from the scope as defined in the appended claims.

## Claims

1. A wearable article (201), comprising at least:
- a flexible support (212);
- an elastic strap (206) attached to the flexible support (212) by first adhesive bond (113) having elastic properties; and
- an elongation sensor (102) sandwiched between the flexible support (212) and the elastic strap (206);
wherein the elongation sensor (102) is attached to the flexible support (212) and/or to the elastic strap (206) by second adhesive bond (114) present along the elongation sensor (102), and
wherein the elongation sensor (102) has electrically conductive properties and is configured to detect, when the wearable article (201) is pressed against a body of a user wearing the wearable article (201), a physiological parameter of the user that is related to an elongation of the elongation sensor (102).

2. The wearable article of claim 1, wherein the first adhesive bond (113) and/or second adhesive bond (114) comprise or comprises an adhesive material (112) disposed between the flexible support (212) and the elastic strap (206), and wherein the elongation sensor (102) is embedded in said adhesive material (112).

3. The wearable article of claim 2, wherein the adhesive material (112) has elastic properties, in such a way that an elongation of the elongation sensor (102) generates an elongation of the adhesive material (112).

4. The wearable article of any one of the preceding claims, wherein the first adhesive bond (113) and/or second adhesive bond (114) is or are created by heat bonding.

5. The wearable article of any one of the preceding claims, wherein the elongation sensor (102) comprises a body of flexible material and conductive particles embedded in the body of flexible material.

6. The wearable article of any one of the preceding claims, wherein the wearable article (201) is a garment comprising a clothing material (202) covering a portion of a body of the user.

7. The wearable article of claim 6, wherein the garment is chosen in a group consisting of a T-Shirt, a belt and a brassiere.

8. The wearable article of any one of the preceding claims, wherein the elongation sensor (102) is configured to be pre-stressed when the wearable article (201) is worn by the user.

9. The wearable article of any one of the preceding claims, further comprising an elastic belt (207), wherein the elongation sensor (102) is located in the elastic belt (207).

10. The wearable article of any one of the preceding claims, further comprising an electronic board (203), wherein the elongation sensor (102) is connected to the electronic board (203).

11. The wearable article of claim 10, further comprising a waterproof housing (204) configured to receive at least the electronic board (203).

12. The wearable article of any one of claims 10 and 11, wherein the elongation sensor (102) has a loop portion (103) at a distance from the electronic board (203), a first section (104) extending from the electronic board (203) to the loop portion (103) and a second section (105) extending from the loop portion (103) back to the electronic board (203).

13. The wearable article of any one of claims 10 - 12, wherein the elongation sensor (102) is connected to the electronic board (203) by at least one conductive yarn (110).

14. The wearable article of claim 13, wherein the wearable article (201) comprises a knotted portion (101), and wherein the elongation sensor (102) is knotted to the at least one conductive yarn (110) in the knotted portion (101).

15. The wearable article of any one of the preceding claims, wherein the elongation sensor (102) is disposed to be located around at least part of an abdomen and/or a chest of the user.
